# EUROPEAN PATENT SPECIFICATION

(11) **EP 3 512 572 B1**
(45) Date of publication and mention of the grant of the patent: **17.07.2024**
(21) Application number: 17768432.1
(22) Date of filing: 13.09.2017
(51) Int. Cl.: A61L 26/00, A61K 9/00, A61L 24/00

(54) **ADHESIVE BIOPOLYMER AS A TEMPORARY EPITHELIUM SUBSTITUTE**
HAFTBIOPOLYMER ALS TEMPORÄRER EPITHELERSATZ
BIOPOLYMÈRE ADHÉSIF EN TANT QUE SUBSTITUT D'ÉPITHÉLIUM TEMPORAIRE

(30) Priority: 16.09.2016 DE 102016217756
(43) Date of publication of application: 24.07.2019
(73) Proprietor: IVIS TECHNOLOGIES S.r.l, 74100 Taranto (IT)
(72) Inventor: D'IPPOLITO, Giuseppe, 74100 Taranto (IT)
(74) Representative: Bardehle Pagenberg Partnerschaft mbB Patentanwälte Rechtsanwälte
(86) International application number: PCT/EP2017/073034
(87) International publication number: WO 2018/050700

(56) References cited:
- WO-A1-2017/132639
- CA-A1- 2 667 708
- US-A- 5 626 863
- SMEDS K A ET AL: "PHOTOCROSSLINKABLE POLYSACCHARIDES FOR IN SITU HYDROGEL FORMATION", JOURNAL OF BIOMEDICAL MATERIALS RESEARCH, WILEY, NEW YORK, NY, US, vol. 54, no. 1, 1 January 2001 (2001-01-01), pages 115 - 121, XP008018719, ISSN: 0021-9304, DOI: 10.1002/1097-4636(200101)54:1<115::AID-JBM14>3.0.CO;2-Q
- PARK Y D ET AL: "Photopolymerized hyaluronic acid-based hydrogels and interpenetrating networks", BIOMATERI, ELSEVIER SCIENCE PUBLISHERS BV., BARKING, GB, vol. 24, no. 6, 1 March 2003 (2003-03-01), pages 893 - 900, XP004399581, ISSN: 0142-9612, DOI: 10.1016/S0142-9612(02)00420-9

## Description

### 1. Field of the invention

The invention relates to the ophthalmic field of biomedical devices. The present invention is directed to an aqueous photo-curable polysaccharide solution with cross-linking properties. Also described is an aqueous photo-curable polysaccharide solution for use in the treatment of a defect in an animal tissue. In particular, the aqueous photo-curable polysaccharide solution is prepared for use in the treatment of a defect in the corneal epithelium and/or stroma. Via in *situ* cross-linking of the aqueous solution, a biocompatible self-adhesive hydrogel is formed that covers the defect, such as a damage or removal of tissue, and acts as a temporary barrier and protective replacement for the tissue.

### 2. Prior art

In some cases, the epithelium, a structure that provides a coating and protection for the internal tissues of the human body, may be damaged or removed by endogenous or exogenous causes.

Generally, in cases of injuries of the corneal epithelial structure, medical devices are applied to the injured part to provide a protective action, such as contact lenses, bandages or products that protect the sub-epithelial structures and stimulate cell regeneration. Injuries of the epithelial tissues may be induced by several causes; they can be particularly troublesome and require specific treatments.

For example, in the ophthalmic field, a total or partial removal of the corneal epithelium is induced by superficial refractive surgery, needed to correct refractive aberrations, such as nearsightedness, farsightedness, astigmatism, due to a deficiency in focusing images on the retina. On the basis of a primary classification, the techniques for refractive surgery may be divided in superficial and intrastromal surgeries. A typical example of a superficial, widely used, refractive surgery, which includes a laser tissue photo ablation, is the PRK (Photo Refractive Keratectomy).

This surface technique envisages a photo ablative removal of tissue, through the use of excimer or solid-state laser with µm accuracy, reshaping the corneal morphology to treat refractive defects. Most recent refractive surgical techniques use a computer controlled laser, so as to drive the photo ablation process with great precision. In a PRK surgery, first the surgeon removes the corneal epithelium through suitable instruments or by means of the alcohol, then he carries out the surgical treatment, by means of the photo ablative laser, or alternatively, in a trans epithelial surgery, the surgeon directly performs, in a single step, by means of the photo ablative laser, the photo ablation of both the epithelium and the stroma.

These surgery interventions are minimally invasive, and are performed under local anesthesia by using anesthetic eye drops, without any need of hospitalization.

Although the technique is simple and fast to carry out, this surgery is subject to unwanted side effects or possible complications.

Once the surgery is completed, generally a protective contact lens is applied, as a bandage, which is then removed during subsequent post-operative visits, when the epithelium has completely regenerated.

The surgery involves occurrence of transient side effects associated with the lack, the damage, total or partial removal of the epithelial structure.

These side effects consist of pain in the eyes, blurred vision, excessive tearing, the feeling of having a foreign body in the eye and photophobia.

Post-surgical complications may even occur when the epithelial structure is damaged or completely or partially removed as haze which causes partial reduction of the stromal transparency and/or star bust effect and /or refractive defect regression. The above listed complication may become permanent and eventually require specific procedures to be addressed.

In the days immediately following the absence, the damage or the total or partial removal of the epithelial structure, the eye remains sore until the epithelium regenerates completely.

Usually, the complete recovery of sight occurs in 2-3 weeks after the event that caused the damage or the total or partial removal of the epithelial structure, while the complete healing process, which depends on an individual biological reaction, sometimes requires a few months.

Therefore, there is a long-felt need for medical devices or other ophthalmic products to act as temporary replacement of the corneal epithelium, when the corneal epithelial structure is damaged, completely or partially absent, such as after a corneal surgery, until the corneal epithelium healing process will be completed. Such replacement should minimize and/or eliminate the above listed undesired transient side effect as pain in the eyes, blurred vision, excessive tearing, the feeling of having a foreign body in the eye and photophobia.

US 5,626,863 A relates to photopolymerizable biodegradable hydrogels for use as tissue adhesives and in controlled drug delivery. CA 2 667 708 A relates to relates to therapeutically useful hydrogel systems. Y.D. Park et al., Biomaterials, 24(6), pages 893-900 (2003), relates to photo polymerized hyaluronic acid-based hydrogels and interpenetrating networks. K.A. Smeds et al., J. Biomed. Mater. Res. 54(1), 115-121 (2001), describes photocrosslinkable polysaccharides for in *situ* hydrogel formation.

### 3. Brief description of the invention

The problem is solved by an aqueous solution according to claim 1.

While hydrogels have previously been used as replacements during wound healing and tissue regeneration (cf. Photo polymerized hyaluronic acid-based hydrogels and interpenetrating networks - Yong Doo Park, Nicola Tirelli, Jeffrey A. Hubbell; Biomaterials 24 (2003) 893-900, Hydrogels in Regenerative Medicine - Brandon V. Slaughter, Shahana S. Khurshid, Omar Z. Fisher, Ali Khademhosseini, and Nicholas A. Peppas; Adv. Mater. 2009, 21, 3307-3329, Designing hydrogel adhesives for corneal wound repair - Mark W. Grinstaff, Biomaterials 28 (2007) 5205-5214, Photopolymerizable hydrogels for tissue engineering applications - Kytai Truong Nguyen, Jennifer L. West, Biomaterials 23, (2002), 4307-4314), no hydrogel has been described to date that is able to form a biocompatible mechanical barrier that is self-adhesive to biological tissue or even the corneal tissue. More in detail, no hydrogel has previously been described that is made from a photo-curable polysaccharide solution to be cross-linked in *situ* in *vivo.* Furthermore, no published studies have indicated or suggested to modulate the stiffness and depolymerization of a hydrogel applied onto the human cornea by application of a hydrolyzing enzyme. The use of, for example, hyaluronidase is known for ophthalmic applications, such as the vitrectomy adjunct application of the vitreous of the eye, as described in US 5,292,509 or US 6,863,886, but not for applications related to healing processes supported by hydrogels.

The present invention provides for a biocompatible hydrogel, which is self-adhesive to biological tissue. A photo-curable polysaccharide is hereafter also referred to as "PPS".

If a photo-initiator in solution is added to the PPS, the resulting solution is also referred to as photo-curable polysaccharide/initiator solution (PPSI). Such PPSI is specifically conceived to be cross-linked in *situ* by means of energy irradiation and, more specifically conceived to be cross-linked in *situ* for ophthalmic application as a temporary replacement of the corneal epithelium.

The inventors have found that such biocompatible hydrogel forms a mechanical barrier, is self-adhesive at the animal tissue, particularly at the corneal stroma, and can act as temporary replacement of the epithelium, when the epithelial structure is absent, damaged, completely or partially removed. The PPSI is specifically conceived to be cross-linked in *situ* to create a temporary self-adhesive barrier to the animal tissue.

The self-adhesive hydrogel on a biological substrate, such as the corneal stroma, performs the following functions: it reduces the pain caused by the mechanical action of the eyelids, reduces the effect of blurred vision, reduces the excessive tearing, minimize the feeling of having a foreign body in the eye, reduces photophobia, defends against attacks of mechanical origin, of the external environment, i.e. chemical, biological and microbiological contamination, lubricates the eye, in that it replicates the action of the mucosal component of the epithelial surface layer. Also, such hydrogel has a high level of transparency thus ensuring visual comfort for the patient.

According to a first embodiment, an aqueous solution comprises at least one photo-curable polysaccharide, and lithium phenyl-2,4,6-trimethylbenzoylphosphinate (LAP) as photo-initiator, wherein the at least one photo-curable polysaccharide is contained in a concentration between 0.1 and 25% w/v, preferably between 0.2 and 20% w/v, more preferably between 0.5 and 15% w/v, and wherein the at least one photo-initiator is contained in a concentration between 0.02 and 0.5% w/v, preferably 0.03 and 0.1% w/v.

The use of LAP is advantageous, since the irradiation time can be as short as a few seconds during the photo-curing reaction. A further advantage is that LAP is non-toxic, such that higher concentrations can be chosen, in comparison to other available photo-initiators. Therefore, by use of LAP, the curing reaction of the photo-curable polysaccharide, e.g. the curing time, can be optimized and specifically adapted to the mode of application.

The polysaccharide is preferably transparent and biocompatible; it may, for example, be selected from either methylcellulose, ethylcellulose, ethylmethylcellulose, hydroxyethylcellulose, hydroxypropylcellulose, ethylhydroxyethylcellulose, hydroxypropylmethylcellulose, carboxymethylcellulose, glycosaminoglycans, or mixtures thereof.

According to a preferred embodiment, the at least one photo-curable polysaccharide has a molecular weight of between 30 kDa and 5 MDa, preferably between 40 kDa and 2.5 MDa, more preferably 50 kDa and 1.25 MDa.

The inventors found that the chain length of the polysaccharide can be varied in anti-correlation with its concentration in order to achieve a suitable viscosity of the aqueous solution for use as a hydrogel. For example, a polysaccharide of 50 kDa molecular weight can be used in a concentration of 5%, and a polysaccharide of 1 MDa molecular weight can be used in a concentration of 1.0%, in order to achieve comparable viscosity. A preferred aqueous solution comprising at least one photo-curable polysaccharide is an aqueous solution having a viscosity of between 50 to 1,000,000 mPa·s (50 to 1,000,000 cP), preferably between (100 and 500,000 mPa·s ((00 and 500,000 cP), more preferably between 1000 and 100,000 mPa·s (1000 and 100,000 cP).

Aqueous solutions of such viscosities are liquid enough to spread on the surface of the tissue while being viscous enough to allow good application on a defect in an animal tissue and cross-linking efficiency.

According to a preferred embodiment, 3 to 70%, preferably 5 to 50%, more preferably 10 to 30% of the saccharide units of the at least one photo-curable polysaccharide exhibit a photo-curable group.

The lower the concentration of the polysaccharide, the higher the functionalization ratio of the polysaccharide with the photo-curable group needs to be in order to achieve a suitable cross-linking efficiency.

Preferably, the photo-curable group is an acrylic group or a derivative thereof such as a methacrylic group.

The inventors have found out that these photo-curable groups are particularly useful for achieving cross-linking of the polysaccharides in order to obtain a hydrogel suitable as a temporary epithelium substitute.

Preferably, the at least one photo-curable polysaccharide is a photo-curable hyaluronic acid.

Hyaluronic acid is particularly useful because it is naturally present in the human body, so it is highly biocompatible and it has regenerative, hydration and lubricative properties. Other preferred polysaccharides are hydrophilic glycosaminoglycans in general.

According to another preferred embodiment, the at least one photo-curable polysaccharide is acrylated and/or methacrylated hyaluronic acid.

According to another preferred embodiment, the aqueous solution further comprises at least one glycosidase.

The glycosidase in the aqueous solution can degrade the photo-curable polysaccharide. Thereby, by combining the photo-curable polysaccharide and the glycosidase in the solution, the stability and life-time of the polysaccharide can be precisely controlled.

The glycosidase can be in particular selected from the group consisting of chondroitinase ABC, chondroitinase AC, chondroitinase B, chondroitin 4-sulfatase, chondroitin 6-sulfatase, hyaluronidase and B-glucuronidase. These enzymes are suitable for degrading photo-curable or photo-cured polysaccharides to smaller oligosaccharides and disaccharides. Most preferably, the glycosidase is a hyaluronidase.

According to a preferred embodiment, the aqueous solution is prepared for cross-linkage with a light source of between 250 and 1600 nm wavelength, preferably between 300 and 850 nm, more preferably between 350 and 650 nm.

Such wavelength ensures good cross-linkage of the aqueous solution to form a biocompatible hydrogel as well as self-adhesion of such hydrogel with the contacted tissue.

Preferably, the aqueous solution is prepared for cross-linkage with a light source at an intensity density of between 0.1 and 100 mW/cm² and an exposure time of between 0.1 seconds and 30 minutes.

The total energy applied by the light source shall not be detrimental to the tissue. Also, the exposure time needs to be acceptable to the patient. Thus, intensity density of the light source needs to be balanced with the exposure time to ensure cross-linkage on the one hand and patient compliance on the other.

Also described is an aqueous solution for use in the treatment of a defect in an animal tissue comprises at least one photo-curable polysaccharide and at least one photo-initiator, wherein the aqueous solution is prepared for use in covering the defect in an animal tissue, wherein the defect in an animal tissue is a defect in a corneal epithelium and/or corneal stroma, wherein the aqueous solution is prepared for in situ cross-linkage, and wherein the aqueous solution is prepared for creating a temporary self-adhesive barrier or to obtain a temporary corneal epithelium substitute..

The inventors have found that such aqueous solution can be used to form a mechanical barrier and to act as temporary replacement of the tissue when the tissue is absent, damaged, completely or partially removed.

According to a preferred embodiment, the aqueous solution is prepared for forming a self-adhesive biocompatible hydrogel on the defect upon in *situ* cross-linkage.

The inventors have found that the resulting self-adhesion of the hydrogel is an advantageous characteristic, since it prevents slippage of said barrier or protective layer over the tissue. Self-adhesion is accomplished, during the in *situ* cross-linking process, by means of the chemical bonds generated between the photo-curable groups activated by the photo-initiators and the amino groups of the collagen fibers of the tissue and by means of a network of weak chemical hydrophilic and hydrophobic bonds between the hydrogel and the collagen components of the tissue. Such self-adhesive hydrogel behaves as a barrier that covers the exposed tissue, in particular human tissue.

Preferably, the aqueous solution for use in the treatment of a defect in an animal tissue is prepared for forming a self-adhesive biocompatible hydrogel having a thickness between 5 and 500 µm, preferably between 10 and 200 µm, more preferably between 20 and 100 µm.

Using less aqueous solution results in a less efficient coverage of the exposed tissue with a reduced barrier function. Too much aqueous solution on the other hand, results in formation of a hydrogel barrier having an excessive thickness that may last for an undesired too long period of time onto the tissue.

Also described is an aqueous solution for use in the treatment of a defect in an animal tissue comprises at least one photo-curable polysaccharide and at least one photo-initiator, wherein the aqueous solution is prepared for use in covering the defect in an animal tissue, wherein the defect in an animal tissue is a defect in a corneal epithelium and/or corneal stroma, wherein the aqueous solution is prepared for in situ cross-linkage, and wherein the aqueous solution is prepared for forming a self-adhesive biocompatible hydrogel having a thickness between 5 and 500 µm, preferably between 10 and 200 µm, more preferably between 20 and 100 µm.

As explained above, the in *situ* cross-linking reaction of the aqueous solution and thus formation of a hydrogel provides for a mechanical barrier, acting for example as a temporary replacement of the tissue when the tissue is absent, damaged, completely or partially removed.

Preferably, the aqueous solution for use in the treatment of a defect in an animal tissue is for use in the treatment of a defect in a human tissue, wherein the defect in an animal tissue is a defect in the human corneal epithelium and/or stroma.

Preferably, the aqueous solution for use in the treatment of a defect in an animal tissue is prepared for depositing or spraying the aqueous solution onto the defect in an animal tissue.

The inventors have found that depositing or spraying the aqueous solution with suitable means allows to achieve a homogenous distribution of the aqueous solution over the whole surface of the exposed tissue, with a regular and preferably constant thickness thus avoiding undesired uneven surfaces which may induce, in case of application onto the corneal stroma, undesired refractive effects.

The defect may be an absent, damaged, completely removed or partially removed corneal epithelium, including or not a corneal stroma removal or a corneal stroma defect. The defect may be caused by a refractive surgery or by a corneal epithelium scraping or by a corneal pathology or by any possible corneal injury. The refractive surgery may be a superficial refractive surgery including laser tissue photo ablation, such as photo refractive keratectomy.

The defect may not be a corneal perforation, partial-thickness laceration or a full-thickness laceration. These kinds of damages can be caused, for example, by infections or sharp elements getting into the eye.

According to a preferred embodiment, the aqueous solution for use in the treatment of a defect in an animal tissue is an aqueous solution according to any of claims 1 to 6.

The aqueous solutions described in claims 1 to 6 is specifically designed and adapted to perform best as a biocompatible hydrogel adhering to animal tissue. The combination of a preferred photo-curable group, a preferred polysaccharide and a preferred photo-initiator generates the capability to obtain, through the in *situ* cross-linking process the optimum self-adhesive biocompatible hydrogel to adhere at the tissue, such as a human tissue.

The inventors have found that, typically, the self-adhesive hydrogel, obtained upon in situ cross-linking reaction mediated by UV or visible light, is compatible with common solution-based eye drugs used in the ophthalmological practice, such as antibiotic, steroidal anti-inflammatory (cortisone), NSAID eye drops, eye drops for glaucoma, artificial tears.

According to another preferred embodiment, the aqueous solution for use is prepared for being supplemented with an enzymatic solution comprising at least one glycosidase following in *situ* cross-linkage.

The inventors have found that the administration of specific enzyme preparations into the eyes of humans or other mammals may help the process of control and modulation of the stiffness, depolymerization, detachment or uncoupling of photo-curated hydrogel bonded to the human corneal epithelium and/or stroma surface. Such uncoupling or depolymerization procedures are purported by enzymatic hydrolysis to precisely modulate the lifetime of the hydrogel onto the corneal surface of the eye and/or to minimize the likelihood that endogenous biodegradation mechanisms of the eye and/or the healing processes of the corneal structure are not sufficient to replace or remove the polysaccharide barrier from the eye.

The at least one glycosidase may be selected from the group consisting of chondroitinase ABC, chondroitinase AC, chondroitinase B, chondroitin 4-sulfatase, chondroitin 6-sulfatase, hyaluronidase and B-glucuronidase.

The at least one glycosidase may be contained in a concentration of between 1 to 5000 U/ml, preferably between 10 and 1000 U/ml, more preferably between 100 and 600 U/ml

The inventor found that the composition of the enzymatic solution can be varied in its composition and concentration in order to achieve a suitable lifetime of the hydrogel replacement by means of the corneal epithelium. For example, native hyaluronidases from different sources, or recombinant version of the enzymes produced through heterologous expression, can hydrolyze the disaccharides at hexosaminidic β (1-4) linkages of hydrogel composed by hyaluronic acid residues. Depending on the cleavage site, hyaluronic fragments size results in either oligosaccharides or other larger polymers. Significant evidence indicates that hyaluronidases derived from different sources differ in enzyme molecular weight distribution and in specific enzymatic activities. Such variability in molecular weight distribution and specific enzymatic activity are noteworthy considerations in view of the fact that hyaluronidase enzymes may be isolated from a variety of sources. In humans, six hyaluronidases have been identified which contribute to this process: HYAL-1, -2, -3, -4, PH-20, and HYALP1. As testicular tissue is a physiological source of bioactive hyaluronidase, bovine or ovine hyaluronidase can be extracted from testes. In addition to humans, hyaluronidases have been found in a variety of venoms from snakes, lizards, and insects, certain bacteria such as streptomyces and certain invertebrate animals such as leeches.

A preferred enzymatic solution to control and to modulate stiffness and depolymerization of the hydrogel in order to achieve a suitable lifetime of the hydrogel replacement by means of the corneal epithelium, comprises at least one glycosidase, preferably a hyaluronidase, is an enzymatic solution having an enzymatic activity of between 1 and 5000 Unit per ml, preferably between 10 and 1000 Unit per ml, more preferably between 100 and 600 Unit per ml.

The enzymatic solution can be deposited or sprayed onto the in *situ* cross-linked aqueous solution one to twenty-four times a day, preferably one to twelve times a day, more preferably one to three times a day. A preferred volume of application is between 5 and 200 µl per each application, preferably between 10 and 100 µl per each application, more preferably between 30 µl and 70 µl per each application.

As utilized in accordance with the present application, the following terms, unless otherwise indicated, shall be understood to have the following meanings:
The term "biocompatible" refers to a substance, which is not toxic and does not cause allergic reactions to the host organism, typically, the human body.

The term "photo-initiator" refers to a molecule that generates reactive species, such as free radicals, when exposed to radiation by UV or visible light.

The term "photo-curable" refers to a chemical group, such as an acrylic group, which is sensitive to reactive species so that the group is activated and reacts with a complementary chemical group to form a bond, such as a covalent bond, in the process of cross-linking.

The term "self-adhesive" in the context of a biocompatible hydrogel refers to a hydrogel, which forms chemical interactions with the biological tissue of interest, such as the human tissue, preferably the corneal stroma. The term "defect in the corneal epithelium" refers to a damage or a lack of the corneal epithelium. Such damage can range in size from a small defect of the tissue structure to a significant gap in the corneal epithelium up to the complete absence of the epithelium on the entire corneal stromal surface.

The term "polysaccharide" refers to a natural or synthetic polysaccharide.

"Hyaluronic acid" refers to repeated disaccharide units composed of D-glucuronic acid and N-acetylglucosamine connected by a glycosidic β1-3 bond. The disaccharide units are connected by a glycosidic β1-4 bond. The structure has the following general formula (C₁₄H₂₁NO₁₁)ₙ 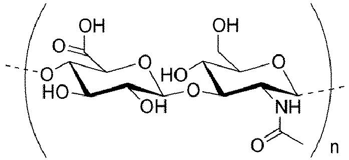 where n is the number of disaccharide units that are repeated along the molecular skeleton of the biopolymer. The hyaluronic acid may have a molecular weight between 30.000 to 5.000.000 Dalton, preferably but not necessarily between 40.000 to 2.500.000 Dalton, more preferably 50.000 to 1.250.000 Dalton. A suitable hyaluronic acid can be as such or in the form of salt, for example sodium hyaluronate. For example, the sodium hyaluronate may be obtained by a recombinant technology using a bacterial fermentation process. Advantageously, during biosynthesis, the microorganism produces a very low quantity of endotoxins (≤ 0.005 IU/mg) and does not produce any exotoxins, thus reducing the risk of toxic contaminations in the extracted and purified hyaluronic acid compared to those gained from animal origin.

"Cross-linking" or "cross-linkage" refers to chemical reactions between specific photo-curable groups.

Regarding the photo-curable polysaccharide, photo-curable groups are first attached to the polysaccharide. For hyaluronic acid, this is usually done via an "esterification reaction".

According to a preferred but not exclusive embodiment, the chemical reagent used for the reaction is the glycidyl methacrylate, in this case the reaction is called "methacrylation reaction" and the photo-curable groups that form the appendices in the structure of hyaluronic acid are the methacrylic groups, according to the following scheme of reaction:

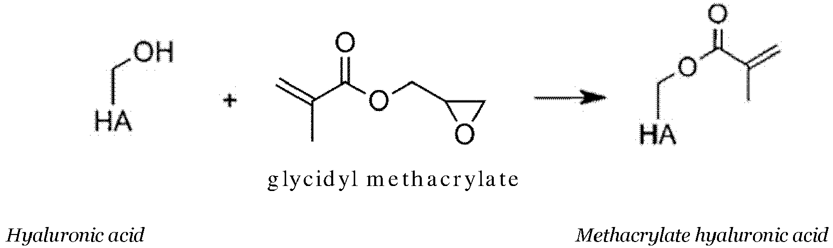

The methacrylic groups are highly reactive and they are activated even in presence of low quantity of photo-initiator when irradiated by UV or visible light at a predefined wavelength and exposure time.

The term "hydrogel" refers to a cross-linked photo-curable solution comprising at least one photo-curable polysaccharide.

In the context of the invention, the term "hyaluronic acid" is considered to include hyaluronic acid as such or in salified form.

The term "animal tissue" is considered to also comprise human tissue. In fact, human tissue is the preferred field of application in the context of the present invention.

The term "glycosidase" refers to enzymes belonging to the enzyme commission number 3.2.1.

The term "hyaluronidase" (EC 3.2.1.35) is commonly used to describe a group of endo-B-glucuronidase enzymes which depolymerize certain mucopolysaccharides, such as hyaluronic acid. Myer, K. et al., The Enzymes; Vol. 4, 2d, Ed., pp 447, Academic Press, Inc., New York (1960). Hyaluronidase causes hydrolysis of the endo-N-acetyl hexosaminic bonds of hyaluronic acid and of the chondroitin sulfate acids A and C, primarily to tetrasaccharide residues.

### 4. Brief description of the drawings

**Fig.1** describes the geometry, the profile and the structure of a cornea in which the epithelial structure is absent, damaged or removed in the central portion.
**Fig. 2** shows the application of the aqueous solution of the invention in the form of an adhesive hydrogel crosslinked in *situ* onto the cornea in which the epithelial structure is absent, damaged, completely or partially removed.
**Fig. 3** shows a methacrylic hyaluronic acid derivative.
**Fig. 4** shows images obtained by Confocal Optical Microscopy, namely LSCM-Laser Scanning Confocal Microscopy:
   a) the deposition of fluorescent polysaccharide solution
   b) the disappearance of fluorescent polysaccharide solution from to corneal stroma after washing with physiological water.
**Fig. 5** shows images obtained by Confocal Optical Microscopy, namely LSCM-Laser Scanning Confocal Microscopy:
   a) the deposition of fluorescent PPSI onto the corneal stroma; after in *situ* cross-linking mediated by UV light.
   b) the persistence of the fluorescent hydrogel onto the corneal stroma after several washes with physiological water.
**Fig. 6** shows a H-NMR spectrum of PPS.
**Fig 7**. shows an image obtained by Scanning Electron Microscopy of an (Fig. 7a) *ex vivo* human corneal stroma with no PPSI application and an *ex vivo* (Fig. 7b) corneal stroma with hydrogel obtained by UV irradiation in *situ* cross-linking of the applied PPSI.
**Fig. 8** shows images obtained by Atomic Force Microscopy of the surfaces of the *ex vivo* human corneal stroma in wet state, before and after application of PPSI and subsequently UV irradiation.
**Fig. 8a** shows the corrugate surface of the corneal stroma deprived of the epithelium.
**Fig. 8b** shows the leveling effect, up to a maximum height difference of 7.2 nm, achieved onto the stroma surface, after application of PPSI and subsequently UV irradiation.
**Fig. 9** shows an image obtained by Laser Scanning Confocal Microscopy of a stromal human tissue treated with an embodiment of fluorescence PPSI application and in *situ* cross-linking. The image is obtained by the superposition of individual frames captured along the observation z axis (Δz= 5 µm).
**Fig. 10** **a** shows a tomographic cross section of an *ex vivo* human cornea deprived of the epithelium after application of PPSI and subsequently UV irradiation.

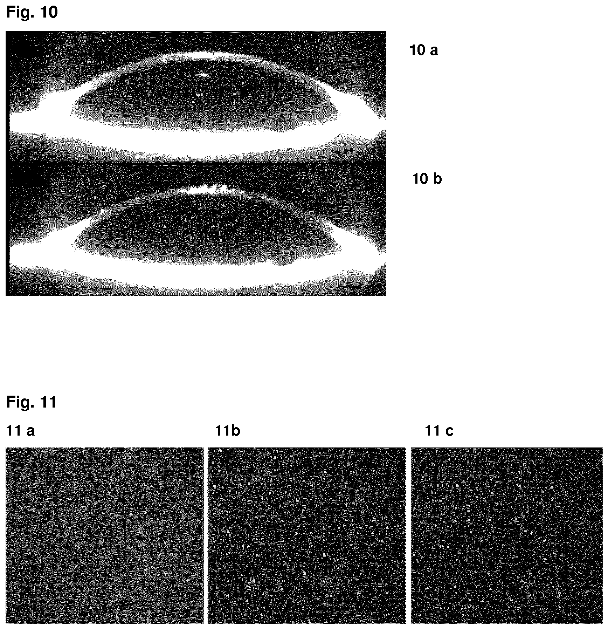

Fig. 10 b shows a tomographic cross-section of a de-epithelialized *ex vivo* human cornea without application of PPSI.
Fig. 11 a shows a stromal human tissue treated with fluorescence PPSI and subsequently UV irradiation.
Fig 11 b and Fig. 11 c, show the permanence of the fluorescence hydrogel after a first and a second mechanical action mediated by a wet ophthalmic lancet.

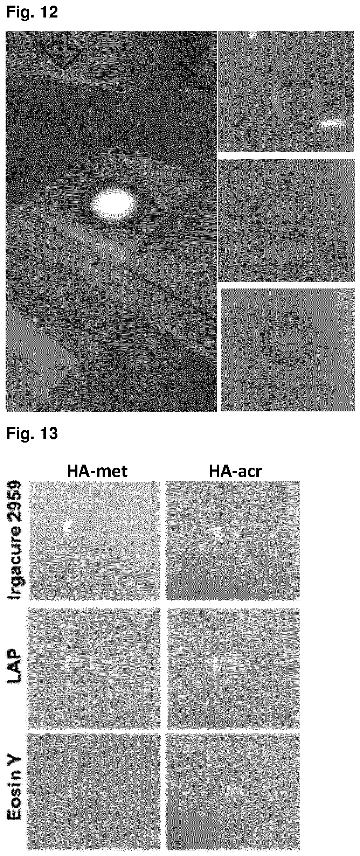

**Fig. 12** shows the cross-linking reaction phases performed by cross-linking UV light.
**Fig. 13** shows hydrogels obtained by means of an in *situ* UV irradiation of the aqueous solution of the invention on glass slides.

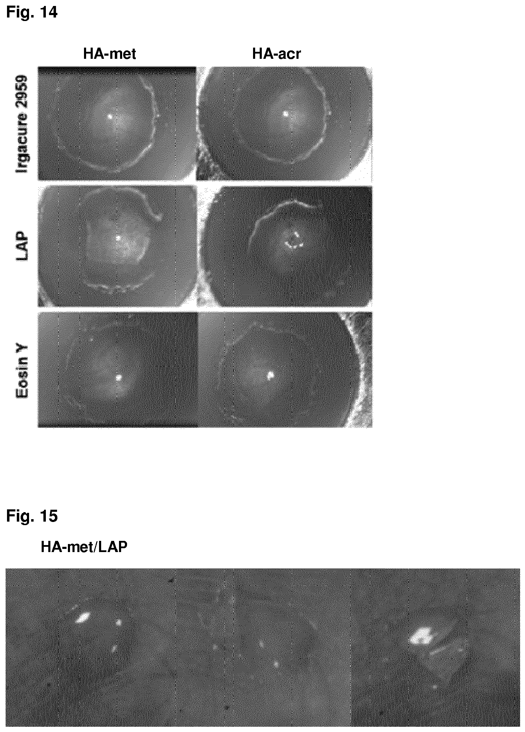

**Fig. 14** shows hydrogels achieved by means of an in *situ* UV irradiation of the aqueous solution of the invention onto the corneal epithelium of cadaver eyes in wet state.
**Fig. 15** shows hydrogels achieved by means of an in *situ* UV irradiation of the aqueous solution of the invention onto the human derma.

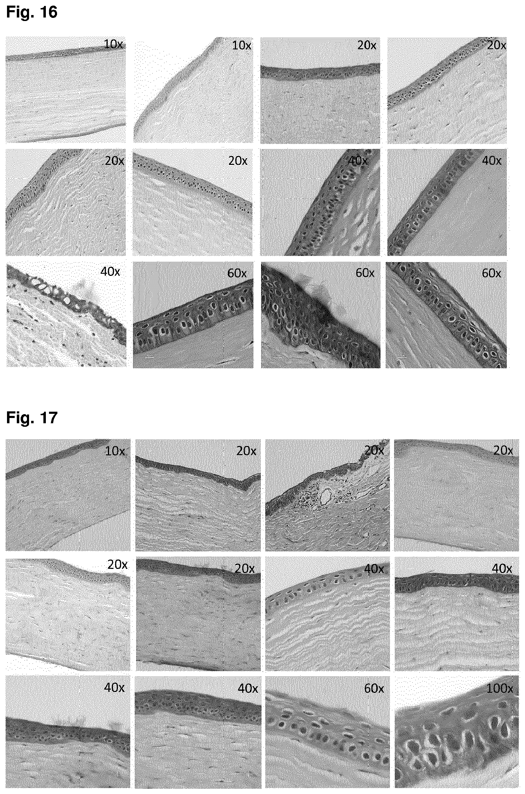

**Fig. 16** shows stained sections using haematoxylin - eosin (15a; 15b; 15d; 15e; 15f; 15g) and stained sections using *Masson's trichrome* (15c; 15h; 15i; 15l; 15m; 15n) of a sacrificed rabbit.
**Fig. 17** shows stained sections using haematoxylin - eosin (16d; 16e; 16g; 16m) and stained sections using *Masson's trichrome* (16a; 16b; 16c; 16f; 16h; 16i; 16l; 16n), of a sacrificed rabbit.

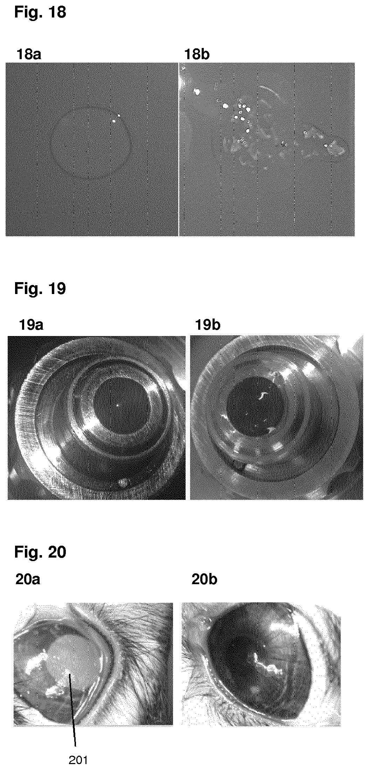

**Fig. 18** **a and b** shows hydrogels achieved by means of an in *situ* UV irradiation of the aqueous solution of the invention on glass slides before and after hyaluronidase enzymatic treatment, respectively.
**Fig. 19** **a and b** shows hydrogels achieved by means of an in *situ* UV irradiation of the aqueous solution of the invention onto the corneal epithelium of cadaver eyes in wet state, before and after hyaluronidase enzymatic treatment, respectively.
**Fig. 20** shows a hydrogel 201 achieved by means of an in *situ* UV irradiation of the aqueous solution of the invention in *vivo* onto the rabbit eyes. **Fig. 20** **a** and **b** show the rabbit eye before hyaluronidase deposition and after 3 days of enzymatic treatment, respectively

### 5. Detailed description of the invention

Currently preferred embodiments of the invention will be described in the following detailed description. It is emphasized, however, that the present invention is not limited to these embodiments.

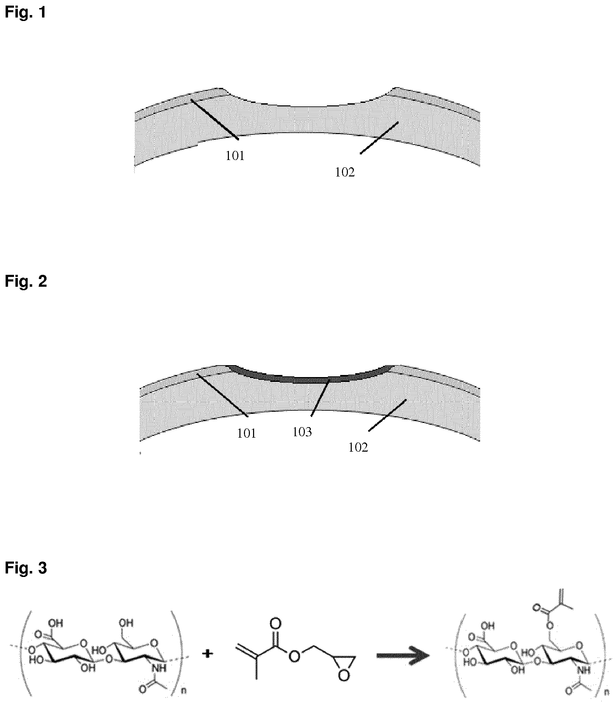

In the ophthalmic field, a total or partial removal of the corneal epithelium is induced by superficial refractive surgery. In this regard, Fig. 1 shows parts of an eye, with the epithelium 101 and the stroma 102, comprising a damage site (illustrated as concave shape). To support the healing process of such damages, the present invention provides for a self-adhesive temporary replacement 103 that can be placed where the epithelial structure 101 (and/or stroma) comprises damages.

As determined experimentally, hyaluronic acid as such, in aqueous solution, has no bioadhesive properties and has no mechanical characteristic when directly applied onto the exposed human tissue, preferably onto the corneal stroma.

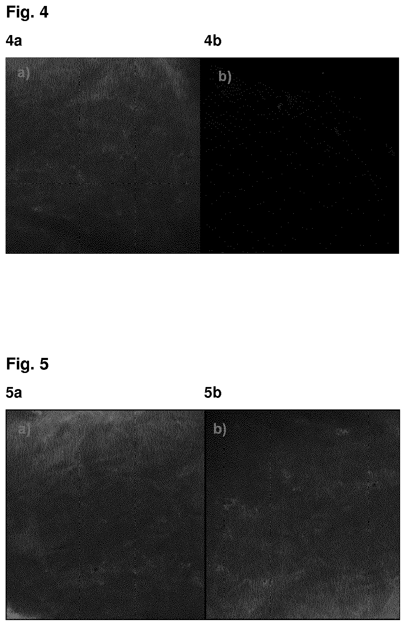

Particularly, it was found that molecules of hyaluronic acid with molecular weights between 30 KDa and 5,0 MDa, fluorescently labeled, do not adhere to corneal stroma. As shown in Fig. 4, hyaluronic acid does not adhere to the stromal corneal surface and is entirely removed by washing with physiological water. This test demonstrates that the hyaluronic acid, as such, has no chemical-physical interaction of any specific type with the collagen of the corneal stroma.

In contrast, photo-curable polysaccharides according to the present invention, such as an aqueous solution comprising methacrylate hyaluronic acid, Fig. 3 have physio-chemical properties that are significantly different from the native polymer, yet being biocompatible.

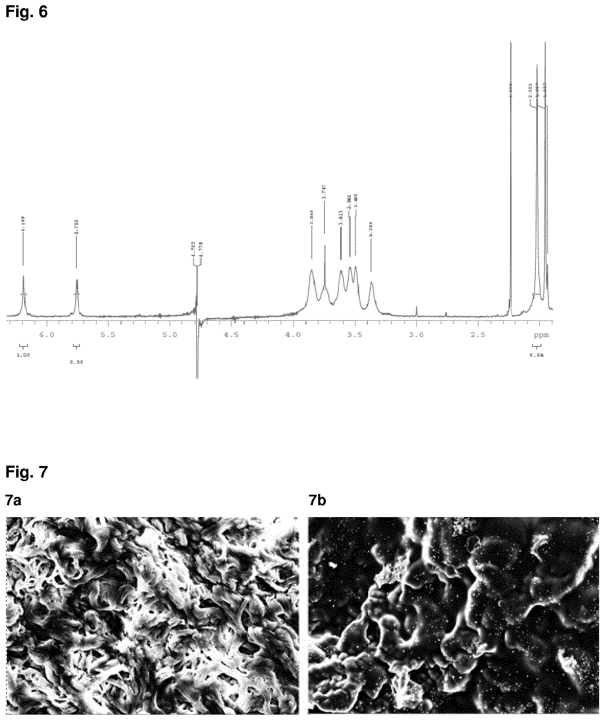

The methacrylate hyaluronic acid used in the examples was determined regarding its degree of methacrylation by H-NMR for all samples of functionalized hyaluronic acid (Fig. 6, NMR Freq.= 600 MHz in D₂O).

The mechanical strength of the cross-linked hydrogel depends on the degree of the functionalization of the photo-curable polysaccharide solution; the higher the functionalization degree, the higher the mechanical strength will be achieved after the cross-linking reaction.

Moreover, a photo-curable hyaluronic acid, in *situ* cross-linked into hydrogel, according to the present invention acts as a stable barrier that adheres at the corneal stromal surface (Fig. 5).

Further preferred embodiments of the present invention are described in the Examples below:

### Example 1

***Preparation of Methacrylate Hyaluronic Acid: Hyaluronic acid esterification with*** glycidyl methacrylate Hyaluronic acid (HA) esterification with glycidyl methacrylate (GM) was performed to obtain methacrylate derivate. It was prepared solving 0.5 g of sodium hyaluronate (CAS number 9067-32-7, HTL Sas, Javene - France, MW= 600 - 1100 KDa) in 100 ml of 0.1 M NaOH (CAS number 1310-73-2, Sigma Aldrich) contained in a round-bottom flask. The mixture was kept under stirring until to complete dissolution. After the dissolution, 1.7 ml of glycidyl methacrylate (GM, CAS number 106-91-2, Sigma Aldrich) was added to the mixture in a dropwise manner.

The reaction was performed in ice cold water at room temperature and pH 8-10 for 6 h. Next, the so achieved methacrylate hyaluronic acid was purified, for one to seven days, by dialysis, using alternatively, each 12 hours, ultrapure water and an acetone, until to remove the unreacted glycidyl methacrylate.

Appropriate membranes (Spectra/Por 4 - Dialysis Membrane, MWCO 12-14 kDa, d= 329 mm,3 0m, Spectrum Laboratories, Inc. - USA) were used for the dialysis. After purification, the methacrylate hyaluronic acid was completely dried under vacuum at room temperature for 12 h in a glass desiccator connected to a high vacuum pump (RC 6 chemistry-HYBRID pump, Vacuubrand, Germany); later on it was stored as white poor at -20 °C until further use.

The presence of methacrylate groups enabled further cross-linking of the HA derivatives, as described in examples 6, 7, 8.

### Example 2

### Preparation of Acrylate Hyaluronic Acid: Hyaluronic acid esterification with acrylic anhydride

Hyaluronic acid (HA) esterification with acrylic anhydride (AA), was performed to obtain acrylate derivate. It was prepared solving 0.5 g of sodium hyaluronate (CAS number 9067-32-7, HTL Sas, Javene - France, MW= 600 - 1100 KDa) in 100 ml of 0.1 M NaOH (CAS number 1310-73-2, Sigma Aldrich) contained in a round-bottom flask. The mixture was kept under stirring until to complete dissolution. After the dissolution, 1.7 ml of acrylic anhydride (AA 90%, CAS number 2051-76-5 Polysciences, Inc) was added to the mixture in a dropwise manner. The reaction was performed in ice cold water at room temperature and pH 8-10 for 6 h.

Next, the so achieved acrylate hyaluronic acid was purified, for one to seven days, by dialysis, using alternatively, each 12 hours, ultrapure water and acetone, until to remove the unreacted acrylic anhydride and acrylic acid.

Appropriate membranes (Spectra/Por 4 - Dialysis Membrane, MWCO 12-14 KDa, d= 329 mm, 30m, Spectrum Laboratories, Inc. - USA) were used for the dialysis. After purification, the acrylate hyaluronic acid was completely dried under vacuum at room temperature for 12 h in a glass desiccator connected to a high vacuum pump (RC 6 chemistry-HYBRID pump, Vacuubrand, Germany); later on it was stored as white poor at -20 °C until further use.

The presence of acrylate groups enabled further -cross-linking of the HA derivatives, as described in examples 9, 10, 11.

### Example 3

### Preparation of Hyaluronic acid labelled with fluorescent probe (HA-fluo)

Carboxylic group of hyaluronic acid (HA) was functionalized with ATTO 647N amine (ATTO-TEC GmbH, Germany as fluorescent probe), namely HA-fluo. The HA-fluo was prepared solving 0.5 g of sodium hyaluronate (CAS number 9067-32-7, HTL Sas, Javene - France, MW= 600 - 1100 KDa) in 100 ml of deionized water contained in a round-bottom flask. The final pH of the solution was adjusted to 6.5 with acid hydrochloride. The mixture was kept under stirring until to complete dissolution. After this time, 0.23 g of N-(3-Dimethylaminopropyl)-N'-ethylcarbodiimide hydrochloride (EDAC, CAS number 25952-53-8, Sigma Aldrich) and 0.14 g of N-Hydroxysuccinimide (NHS 98%, CAS number 6066-82-6, Sigma Aldrich) were added to hyaluronic acid solution and the mixture was kept under stirring for 10 minutes.

Next, 0.5 mg of ATTO-647N-NH₂ (ATTO-TEC GmbH, Germany) previously activated with 20 µl of Triethylamine (TEA 99%, CAS number 121-44-8, Sigma Aldrich) was added to hyaluronic acid solution.

The reaction was performed at room temperature and pH 6.8 for 4 h. The HA-fluo was purified for two days, by dialysis, using ultrapure water, until to remove unreacted ATTO-647N-NH₂ by-product. Appropriate membranes (Spectra/Por 4 - Dialysis Membrane, MWCO 12-14 KDa, d= 329 mm, 30m, Spectrum Laboratories, Inc. - USA) were used for the dialysis.

After purification, the HA-fluo was completely dried in two steps:
a) precipitation in acetone (CAS number 67-64-1, Sigma Aldrich)
b) under vacuum at room temperature for 8 h in a glass desiccator connected to a high vacuum pump (RC 6 chemistry-HYBRID pump, Vacuubrand, Germany), and stored as fluorescence poor at -20 °C until further use.

The final product was characterized by FTIR and Confocal Optical Microscopy (LSCM - Laser Scanning Confocal Microscopy).

### Example 4

***Preparation of Fluorescence Methacrylate Hyaluronic acid: HA-fluo esterification with*** glycidyl methacrylate Hyaluronic acid labelled with a fluorescent probe (HA-fluo) esterification with glycidyl methacrylate (GM) was performed to obtain fluorescence methacrylate hyaluronic acid. It was prepared solving 0.4 g of HA-fluo in 80 ml of 0.1 M NaOH (CAS number 1310-73-2, Sigma Aldrich) contained in a round-bottom flask. The mixture was kept under stirring until to complete dissolution. After the dissolution, 1.7 ml of glycidyl methacrylate (GM, CAS number 106-91-2, Sigma Aldrich) were added to the mixture in a dropwise manner.

The reaction was performed in ice cold water at room temperature and pH 8-10 for 6 h. Next, the so achieved fluorescence methacrylate hyaluronic acid was purified, for one to seven days, by dialysis, using alternatively, each 12 hours, ultrapure water and acetone, until to remove the unreacted glycidyl methacrylate.

Appropriate membranes (Spectra/Por 4 - Dialysis Membrane, MWCO 12-14 kDa, d= 329 mm, 30m, Spectrum Laboratories, Inc. - USA) were used for the dialysis.

After purification, the fluorescence methacrylate derivate was completely dried under vacuum at room temperature for 12 h in a glass desiccator connected to a high vacuum pump (RC 6 chemistry-HYBRID pump, Vacuubrand, Germany), and later on it was stored as fluorescence poor at -20 °C until further use.

The final product was characterized by FTIR and Confocal Optical Microscopy (LSCM - Laser Scanning Confocal Microscopy).

### Example 5

### Preparation of PPSI using Fluorescence Methacrylate Hyaluronic Acid and LAP photo-initiator

The fluorescent methacrylate hyaluronic acid product obtained via glycidyl methacrylate was further cross-linked by free-radical polymerization when subjected to UV-light (365 nm) and a photo-initiator such as lithium phenyl-2,4,6-trimethylbenzoylphosphinate (LAP, CAS number 85073-19-4, Tokyo Chemical Industry).

5.0% fluorescence PPSI was prepared using 50 mg of fluorescence methacrylate hyaluronic acid dissolved in 1 ml of 0,05% LAP water solution until completely dissolution.

### Example 6 (not according to the invention)

### Preparation of Hydrogel via cross-linking reaction using a PPSI (Methacrylate Hyaluronic Acid and Irgacure 2959 photo-initiator)

The Methacrylate Hyaluronic Acid obtained via glycidyl methacrylate was further crosslinked by free-radical polymerization when subjected to UV-light (365 nm) and a photo-initiator such as 2-hydroxy-4'-(2-hydroxyethoxy) 2-methylpropiophenone (Irgacure 2959, CAS number 106797-53-9, Sigma Aldrich).

5.0% of PPSI was prepared using 50 mg of Methacrylate Hyaluronic Acid dissolved in 1 ml of 0,05% Irgacure 2959 water solution. After completely dissolution, 20 µl of PPSI was disposed on glass slide in the ring having diameter 9 mm (Fig. 13) and exposed to UV-light for 10 minutes at 20 mW/cm².

The transparency is an advantageous characteristic of the self-adhesive hydrogel when applied onto the corneal stroma to allow a good quality of vision for the patient. The transparency is the physical property which enables the light to pass through the material. The measure of the transparency of the so prepared hydrogel was carried out by measuring the refractive index through a single 589.3 nm wavelength sodium refractometer measurement (CLR 12-70 Contact Lens Refractometer, Index Limited Instruments, UK). The refractive index (RI) was measured between 98.5 - 99.9 % compared to that of ultrapure water at 25 °C.

### Example 7

### Preparation of Hydrogel via cross-linking reaction using a PPSI (Methacrylate Hyaluronic Acid and LAP photo-initiator)

The Methacrylate Hyaluronic Acid obtained via glycidyl methacrylate was further cross-linked by free-radical polymerization when subjected to UV-light (365 nm) and a photo-initiator such as lithium phenyl-2,4,6-trimethylbenzoylphosphinate (LAP, CAS number 85073-19-4, Tokyo Chemical Industry).

5.0% of PPSI was prepared using 50 mg of Methacrylate Hyaluronic Acid dissolved in 1 ml of 0,05% LAP water solution. After completely dissolution, 20 µl of PPSI was disposed on glass slide in the ring having diameter 9 mm (Fig. 13) and exposed to UV-light for 5 minutes at 20 mW/cm².

### Example 8 (not according to the invention)

### Preparation of Hydrogel via cross-linking reaction using a PPSI (Methacrylate Hyaluronic Acid and Eosin Y photo-initiator)

The Methacryled Hyaluronic Acid obtained via glycidyl methacrylate was further cross-linked by free-radical polymerization when subjected to UV-light (365 nm) and a photo-initiator such as Eosin Y (EY, CAS number 15086-94-9, Sigma Aldrich).

4.0% PPSI was prepared using 40 mg of Methacrylate Hyaluronic Acid dissolved in 1 ml of 0.5 mM Eosin Y water solution.

The Eosin Y water solution was preliminarily prepared mixing 3,24 mg of Eosin Y in 10 ml of 0.1 M HEPES solution, next adding at 9,86 ml of eosin solution 10 µl of 1-vinyl-2-Pyrrolidone (CAS number 88-12-0, Sigma Aldrich) and 133 µl of Triethanolamine (CAS number 102-71-6, Sigma Aldrich).

After completely dissolution, 20 µl of PPSI was disposed on glass slide in the ring having diameter 9 mm (Fig. 13) and exposed to UV-light for 10 minutes at 20 mW/cm².

### Example 9 (not according to the invention)

### Preparation of Hydrogel via cross-linking reaction using a PPSI (Acrylate Hyaluronic Acid and Irgacure 2959 photo-initiator)

The Acrylate Hyaluronic Acid obtained via acrylic anhydride was further cross-linked by free-radical polymerization when subjected to UV-light (365 nm) and a photo-initiator such as 2-hydroxy-4'-(2-hydroxyethoxy) 2-methylpropiophenone (Irgacure 2959, CAS number 106797-53-9, Sigma Aldrich).

5.0% PPSI was prepared using 50 mg of Acrylate Hyaluronic Acid dissolved in 1 ml of 0,05% Irgacure 2959 water solution.

After completely dissolution, 20 µl of PPSI was disposed on glass slide in the ring having diameter 9mm (Fig. 13) and exposed to UV-light for 10 minutes at 20 mW/cm².

### Example 10

### Preparation of Hydrogel via cross-linking reaction using a PPSI (Acrylate Hyaluronic Acid and LAP photo-initiator)

The Acrylate Hyaluronic Acid obtained via acrylic anhydride was further cross-linked by free-radical polymerization when subjected to UV-light (365 nm) and a photo-initiator such as lithium phenyl-2,4,6-trimethylbenzoylphosphinate (LAP, CAS number 85073-19-4, Tokyo Chemical Industry).

5.0% PPSI was prepared using 50 mg of Acrylate Hyaluronic Acid dissolved in 1 ml of 0,05% LAP water solution.

After completely dissolution, 20 µl of PPSI was disposed on glass slide in the ring having diameter 9mm (Fig. 13) and exposed to UV-light for 5 minutes at 20 mW/cm².

### Example 11 (not according to the invention)

### Preparation of Hydrogel via cross-linking reaction using a PPSI (Acrylate Hyaluronic Acid and Eosin Y photo-initiator)

The acrylate hyaluronic acid obtained via acrylic anhydride was further cross-linked by free-radical polymerization when subjected to UV-light (365 nm) and a photo-initiator such as Eosin Y (EY, code number 15086-94-9, Sigma Aldrich).

4.0% PPSI was prepared using 40 mg of acrylate hyaluronic acid dissolved in 1 ml of 0.5 mM Eosin Y water solution. The Eosin Y water solution was initially prepared mixing 3,24 mg of Eosin Y in 10 ml of 0.1 M HEPES solution, next adding at 9,86 ml of eosin solution 10 µl of 1-vinyl-2-Pyrrolidone (CAS number 88-12-0, Sigma Aldrich) and 133 µl of Triethanolamine (CAS number 102-71-6, Sigma Aldrich).

After completely dissolution, 20 µl of PPSI was disposed on glass slide (Fig. 13) in the ring having diameter 9 mm and exposed to UV-light for 10 minutes at 20 mW/cm².

### Example 12 (not according to the invention)

### Cross-linking reaction of PPSI on ex vivo stromal human cornea sample (using Methacrylate Hyaluronic Acid and Irgacure 2959 photo-initiator)

Human cornea has been laid down and locked on appropriate support system as an artificial chamber, (Fig. 14, Ligi Tecnologie Medicali S.r.l.) to imitate the corneal eye profile like in vivo condition. In this way, the cornea was leaded to pressure using physiological water at 37°C injected with a normal syringe. The epithelium was removed by refractive surgery (Photo Refractive Keratectomy, PRK) using excimer laser (E= 1165 +/- 140 mW, Freq.= 1000 Hz), by Ivis suite platform (Ivis Technologies S.r.l., Taranto, Italy).

The used keratectomy procedure included the following parameters: photo-ablation diameter was of 9 mm, the photo-ablation depth was of 100-120 µm.

Successively the keratectomized cornea was washed with physiological water and dried with ophthalmic lancet. 20 µl of sterile PPSI were deposed on cornea surface and exposed to UV-light for 10 minutes at 20 mW/cm².

Applied PPSI: 5.0% PPSI was prepared using 50 mg of methacrylate hyaluronic acid and dissolved in 1 ml of 0,05% Irgacure 2959 (CAS number 106797-53-9, Sigma Aldrich) water solution until completely dissolution.

### Example 13

### Cross-linking reaction of PPSI on ex vivo stromal human cornea sample (using Methacrylate Hyaluronic Acid and LAP photo-initiator)

Human cornea has been laid down and locked on appropriate support system as an artificial chamber, (Fig. 14, Ligi Tecnologie Medicali S.r.l.) to imitate the corneal eye profile like in vivo condition. In this way, the cornea was leaded to pressure using physiological water at 37°C injected with a normal syringe. The epithelium was removed by refractive surgery (Photo Refractive Keratectomy, PRK) using excimer laser (E= 1165 +/- 140 mW, Freq.= 1000 Hz), by Ivis suite platform (Ivis Technologies S.r.l., Taranto, Italy).

The used keratectomy procedure included the following parameters: photo-ablation diameter was of 9 mm, the photo-ablation depth was of 100-120 µm.

Successively the keratectomized cornea was washed with physiological water and dried with ophthalmic lancet. 20 µl of sterile PPSI were deposed on cornea surface and exposed to UV-light for 5 minutes at 20 mW/cm².

Applied PPSI: 5.0% PPSI was prepared using 50 mg of methacrylate hyaluronic acid and dissolved in 1 ml of 0,05% LAP (CAS number 85073-19-4, Tokyo Chemical Industry) water solution until completely dissolution.

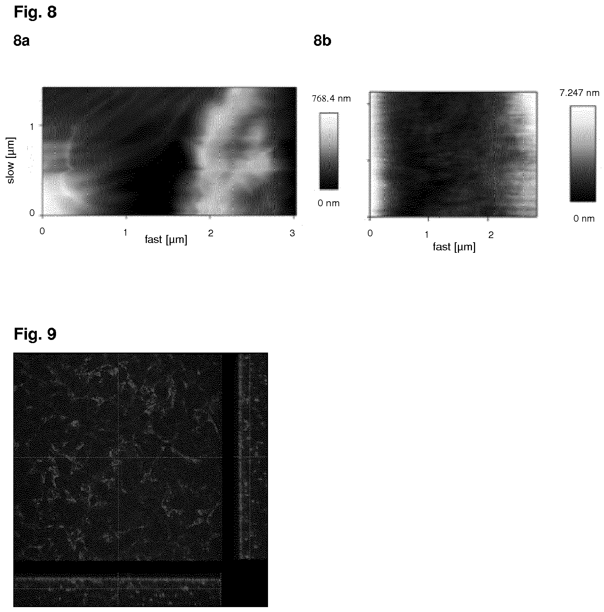

A treated sample of ex vivo human cornea, so prepared, has been subjected to analysis by Scanning Electron Microscopy (SEM, Fig. 7); another sample of cornea instead was analysed by Atomic Force Microscopy (AFM, Fig. 8).

This type of test has allowed us to verify both the behaviour and the effectiveness of the hydrogel. In fact, compared to an untreated control, the presence was observed, through the SEM test, of an exogenous material that covered completely the stromal tissue after in situ cross-linking reaction, preferably but not necessarily, mediated by UV rays, as per in the example 7). AFM images instead confirmed a levelling of the corneal surface treated with PPSI, naturally after in *situ* cross-linking reaction mediated by UV rays, unlike the untreated control.

In two separate tests, fluorescence PPSI, as per example 5, and PPSI, as per example 7, have been applied onto the human corneal stromal surface devoid of the epithelium. Thereafter, the treated corneal samples were subjected to vigorous washing with physiological water at 37 °C. For the preparation of the fluorescence PPSI, the test was performed using Confocal Microscopy to verify the presence and uniformity of coverage of the formed hydrogel, Fig. 9).

According to the tests, the treated sample with fluorescence PPSI after in *situ* cross-linking reaction mediated by UV rays, shows a uniform fluorescence in the area of the de-epithelialized stroma, while the fluorescence is completely absent in the sample which was not treated with fluorescence PPSI. The three-dimensional representation of the thickness of the treated cornea with fluorescence PPSI shows the thickness originated by depositing the fluorescence of the hydrogel.

The test of adhesion the hydrogel, obtained as per example 7, and the stromal collagen was performed using a laminar flow room. The laminar flow simulates the action of blinking of the eyelids, and through this test the mechanical stability of the hydrogel onto the surface of the corneal stromal region can be evaluated.

The PPSI was applied onto a diskette of a human cornea devoid of the epithelium and after was carried in *situ* a cross-linking reaction mediated by UV light.

The test was conducted placing the so prepared diskette in a spherical housing in the centre of the laminar flow room, having it completely closed and sealed. The system was subjected to a laminar flow of physiological water with flow rate of 30 ml per minute, in cycles of 5 minutes each.

After a series of 5 minute cycles, the findings indicated that the hydrogel was resulted firmly adherent to the corneal surface in question.

In addition, to simulate the mechanical action of rubbing of the eyelids (blinking) the cornea treated with fluorescence PPSI, and after in *situ* cross-linking reaction mediated by UV rays, as in example 5, has been subjected to simulation of blinking using a wet ophthalmic lancet. Considering that complete blinking corresponds to a double continuous passage of the wet ophthalmic lancet, simulation has been performed by reproducing 120 full blinking actions in 6 minutes. The fluorescence hydrogel was found stably anchored to the surface of the corneal stroma even after this mechanical action, as shown by LSCM analysis (Fig. 11).

Similarly, to the non-fluorescent hydrogel, as per example 7, the test was performed using the computerized corneal mapping technique (Corneal Topographer, Fig. 10). The topography test has detected the presence of hydrogel thanks to the absence of the sparkling light effect typical of the corneas without epithelium.

### Example 14 (not according to the invention)

### Cross-linking reaction of PPSI on ex vivo stromal human cornea sample (using Acrylate Hyaluronic Acid and Irgacure 2959 photo-initiator)

Human cornea has been laid down and locked on appropriate support system as an artificial chamber, (Fig. 14, Ligi Tecnologie Medicali S.r.l.) to imitate the corneal eye profile like in vivo condition. In this way, the cornea was leaded to pressure using physiological water at 37°C injected with a normal syringe.

The epithelium was removed by refractive surgery (Photo Refractive Keratectomy, PRK) using excimer laser (E= 1165 +/- 140 mW, Freq.= 1000 Hz), by Ivis suite platform (Ivis Technologies S.r.l., Taranto, Italy). The used keratectomy procedure included the following parameters: photo-ablation diameter was of 9 mm, the photo-ablation depth was of 100-120 µm.

Successively the keratectomized cornea was washed with physiological water and dried with ophthalmic lancet. 20 µl of sterile PPSI were deposed on cornea surface and exposed to UV-light for 10 minutes at 20 mW/cm².

Applied PPSI: 5.0% PPSI was prepared using 50 mg of Acrylate Hyaluronic Acid and dissolved in 1 ml of 0,05% Irgacure 2959 (CAS number 106797-53-9, Sigma Aldrich) water solution until completely dissolution.

### Example 15

### Cross-linking reaction of PPSI on ex vivo stromal human cornea sample (using Acrylate Hyaluronic Acid and LAP photo-initiator)

Human cornea has been laid down and locked on appropriate support system as an artificial chamber, (Fig. 14, Ligi Tecnologie Medicali S.r.l.) to imitate the corneal eye profile like in vivo condition. In this way, the cornea was leaded to pressure using physiological water at 37°C injected with a normal syringe. The epithelium was removed by refractive surgery (Photo Refractive Keratectomy, PRK) using excimer laser (E= 1165 +/- 140 mW, Freq.= 1000 Hz), by Ivis suite platform (Ivis Technologies S.r.l., Taranto, Italy).

The used keratectomy procedure included the following parameters: photo-ablation diameter was of 9 mm, the photo-ablation depth was of 100-120 µm.

Successively the keratectomized cornea was washed with physiological water and dried with ophthalmic lancet. 20 µl of sterile PPSI were deposed on cornea surface and exposed to UV-light for 5 minutes at 20 mW/cm².

Applied PPSI: 5.0% PPSI was prepared using 50 mg of Acrylate Hyaluronic Acid and dissolved in 1 ml of 0,05% LAP (CAS number 85073-19-4, Tokyo Chemical Industry) water solution until completely dissolution.

### Example 16 (not according to the invention)

### Cross-linking reaction of PPSI on ex vivo stromal human cornea sample (using Methacrylate Hyaluronic Acid and Eosin Yphoto-initiator)

Human cornea has been laid down and locked on appropriate support system as an artificial chamber, (Fig. 14, Ligi Tecnologie Medicali S.r.l.) to imitate the corneal eye profile like in vivo condition. In this way, the cornea was leaded to pressure using physiological water at 37 °C injected with a normal syringe. The epithelium was removed by refractive surgery (Photo Refractive Keratectomy, PRK) using excimer laser (E= 1165 +/- 140 mW, Freq.= 1000 Hz), Ivis suite platform (Ivis Technologies S.r.l., Taranto, Italy).

The used keratectomy procedure included the following parameters: photo-ablation diameter was of 9 mm, the photo-ablation depth was of 100-120 µm. Successively the keratectomized cornea was washed with physiological water and dried with ophthalmic lancet. 20 µl of sterile PPSI solution were deposed on cornea surface and exposed to UV-light for 10 minutes at 20 mW/cm².

Applied PPSI: 4.0% PPSI was prepared using 40 mg of methacrylate hyaluronic acid dissolved in 1 ml of 0.5 mM Eosin Y (CAS number 15086-94-9, Sigma Aldrich) water solution until completely dissolution.

The Eosin Y water solution was initially prepared mixing 3,24 mg of Eosin Y in 10 ml of 0.1 M HEPES solution, next adding at 9,86 ml of eosin solution 10 µl of 1-vinyl-2-Pyrrolidone (CAS number 88-12-0, Sigma Aldrich) and 133 µl of Triethanolamine (CAS number 102-71-6, Sigma Aldrich) until completely dissolution.

### Example 17 (not according to the invention)

### Cross-linking reaction of PPSI on ex vivo stromal human cornea sample (using Acrylate Hyaluronic Acid and Eosin Y photo-initiator)

Human cornea has been laid down and locked on appropriate support system as an artificial chamber, (Fig. 14, Ligi Tecnologie Medicali S.r.l.) to imitate the corneal eye profile like in vivo condition. In this way, the cornea was leaded to pressure using physiological water at 37 °C injected with a normal syringe. The epithelium was removed by refractive surgery (Photo Refractive Keratectomy, PRK) using excimer laser (E= 1165 +/- 140 mW, Freq.= 1000 Hz), Ivis suite platform (Ivis Technologies S.r.l., Taranto, Italy).

The used keratectomy procedure included the following parameters: photo-ablation diameter was of 9 mm, the photo-ablation depth was of 100-120 µm. Successively the keratectomized cornea was washed with physiological water and dried with ophthalmic lancet. 20 µl of sterile PPSI were deposed on cornea surface and exposed to UV-light for 10 minutes at 20 mW/cm².

Applied PPSI: 4.0% PPSI was prepared using 40 mg of acrylate hyaluronic acid dissolved in 1 ml of 0.5 mM Eosin Y (CAS number 15086-94-9, Sigma Aldrich) water solution until completely dissolution.

The Eosin Y water solution was initially prepared mixing 3,24 mg of Eosin Y in 10 ml of 0.1 M HEPES solution, next adding at 9,86 ml of eosin solution 10 µl of 1-vinyl-2-Pyrrolidone (CAS number 88-12-0, Sigma Aldrich) and 133 µl of Triethanolamine (CAS number 102-71-6, Sigma Aldrich) until completely dissolution.

### Example 18

### Cross-linking reaction of PPSI on human derma (using Methacrylate Hyaluronic Acid and LAP photo-initiator)

Human derma was washed with physiological water and dried with ophthalmic sponge. 20 µl of sterile PPSI were deposed on human tissue surface and exposed to UV-light for 5 minutes at 20 mW/cm² (Fig. 15).

Applied PPSI: 5.0% PPSI was prepared using 50 mg of methacrylate hyaluronic acid and dissolved in 1 ml of 0,05% LAP (CAS number 85073-19-4, Tokyo Chemical Industry) water solution until completely dissolution.

### Example 19

### Cross-linking reaction of PPSI in vivo stromal rabbit cornea (using Methacrylate Hyaluronic Acid and LAP photo-initiator)

In addition, to evaluate the possible cytotoxicity of the hydrogel, the cornea of a rabbit was treated with an embodiment of sterilized PPSI, as in example 7, to perform an in *vivo* test.

Once the rabbit was anesthetized, the epithelium was manually removed. Successively the debrided cornea was washed with physiological water and dried with ophthalmic lancet. After this preliminary operation, 100 µl of sterile PPSI, as per the previous example 7, were uniformly displaced onto the cornea surface and exposed to UV-light for 5 minutes at 20 mW/cm². After the cross-linking reaction, the rabbit was kept under observation for 15 days and the regrowth of the epithelium was daily monitored until complete expulsion of the hydrogel and complete regeneration of the epithelium within seven days.

The result of the histological analysis (Fig. 16) of the cornea of the rabbit showed that:
a) the regenerated epithelium was in its physiological configuration;
b) the epithelial cells were well organized;
c) the hydrogel was completely eliminated from the site of deposition.

### Example 20 (not according to the invention)

### Cross-linking reaction of PPSI on vivo stromal rabbit cornea (using Methacrylate Hyaluronic Acid and Eosin Y photo-initiator)

In addition, to evaluate the possible cytotoxicity of the hydrogel, a cornea of a rabbit was treated with an embodiment of sterile PPSI, as in example 8, to perform an in vivo test.

Once the rabbit was anesthetized, the epithelium was manually removed. Successively the debrided cornea was washed with physiological water and dried with ophthalmic lancet. After this preliminary operation, 100 µl of sterile PPSI, as per the previous example 8, were uniformly displaced onto the cornea surface and exposed to UV-light for 10 minutes at 20 mW/cm².

After the cross-linking reaction, the rabbit was kept under observation for 15 days and the regrowth of the epithelium was daily monitored until complete expulsion of the hydrogel and complete regeneration of the epithelium within seven days.

The result of the histological analysis (Fig.17) of the cornea of the rabbit showed that:
a) the regenerated epithelium was in its physiological configuration;
b) the epithelial cells were well organized;
c) the hydrogel was completely eliminated from the site of deposition.

### Example 21

### Preparation of an enzymatic solution containing hyaluronidase activity for depolymerization of cross-linked hydrogel achieved through PPSI (Methacrylate Hyaluronic Acid and LAP photo-initiator)

The Methacrylate Hyaluronic Acid obtained via glycidyl methacrylate was further cross-linked by free-radical polymerization when subjected to UV-light (365 nm) and the photo-initiator lithium phenyl-2,4,6-trimethylbenzoylphosphinate (LAP, CAS number 85073-19-4, Tokyo Chemical Industry).

7.0% of PPSI was prepared using 70 mg of Methacrylate Hyaluronic Acid dissolved in 1 ml of 0,065% LAP water solution. After completely dissolution, 5 µl of PPSI was disposed on a glass slide in a ring having diameter 9 mm and exposed to UV-light for 1 minute at 18 mW/cm² (Fig. 18 a).

After completion of the polymerization procedure, an enzymatic solution of hyaluronidase (pharmaceutical grade, Jaluran^{R}, Pfizer Italia Srl) containing 300 U per ml, dissolved in physiological solution (0.9 % NaCl in distilled water - weight/volume), was applied on the hydrogel surface. After 3 days of incubation the hydrogel was almost completely depolymerized (Fig. 18 b).

### Example 22

### Preparation of an enzymatic solution containing hyaluronidase activity for depolymerization of cross-linked PPSI on ex vivo stromal human cornea sample (using Methacrylate Hyaluronic Acid and LAP photo-initiator)

Human cornea was laid down and locked onto an appropriate support system as an artificial chamber (see example 13). The epithelium was removed by refractive surgery (Photo Refractive Keratectomy, PRK) using excimer laser (E= 1165 +/- 140 mW, Freq.= 1000 Hz), by means of the iVis Suite platform (Ivis Technologies S.r.l., Taranto, Italy). The used keratectomy procedure included the following parameters: photo-ablation diameter: 9 mm, the photo-ablation depth: 120 µm.

7.0% of PPSI was prepared using 70 mg of Methacrylate Hyaluronic Acid dissolved in 1 ml of 0.065% LAP (CAS number 85073-19-4, Tokyo Chemical Industry) water solution until completely dissolution.

The keratectomized cornea was washed with physiological water and dried with an ophthalmic lancet. 5 µl of sterile PPSI were deposed on cornea surface and exposed to UV-light for 1 minute at 18 mW/cm² (Fig. 19a). After completion of the polymerization procedure, an enzymatic solution of hyaluronidase (pharmaceutical grade, Jaluran^{R}, Pfizer Italia Srl) containing 300 U per ml, dissolved in physiological solution (0,9% NaCl in distilled water - weight/volume), was applied on the hydrogel surface. After 3 days of incubation the hydrogel was almost completely depolymerized (Fig. 19b).

### Example 23

### Preparation of an enzymatic solution containing hyaluronidase activity for depolymerization of in vivo cross-linked PPSI on stromal rabbit cornea (using Methacrylate Hyaluronic Acid and LAP photo-initiator)

Once the rabbit was anesthetized, the epithelium was removed by refractive surgery (Photo Refractive Keratectomy, PRK) using excimer laser (E= 1165 +/- 140 mW, Freq.= 1000 Hz), by means of the iVis Suite platform (Ivis Technologies S.r.l., Taranto, Italy). The keratectomy procedure included the following parameters: photo-ablation diameter: 9 mm, the photo-ablation depth: 120 µm. Following the keratectomy, the keratectomized cornea was washed with physiological water and dried with an ophthalmic lancet. A volume of 5 µl of sterile PPSI, prepared as described in example 22, was uniformly displaced onto the cornea surface and exposed to UV-light for 1 minute at 18 mW/cm² (Fig. 20 a). The hydrogel 201 was coloured in green by addition of a fluorescein solution. After completion of the cross-linking reaction, a deposition of an enzymatic solution of hyaluronidase pharmaceutical grade (Jaluran^{R}, Pfizer Italia Srl) containing 600 U per ml dissolved in physiological solution (0,9% NaCl in distilled water - weight/volume) was applied onto the hydrogel surface in the amount of one drop (approximately 40 µl), two times per day. The rabbit was kept under observation for 15 days, the hydrogel presence was daily monitored until almost complete depolymerization of the hydrogel which occurred within three days (Fig. 20 b) versus the depolymerization of example 20 which occurred within seven days.

## Claims

1. An aqueous solution comprising:
(i) at least one photo-curable polysaccharide, and
(ii) lithium phenyl-2,4,6-trimethylbenzoylphosphinate (LAP) as photoinitiator,
wherein the at least one photo-curable polysaccharide is contained in a concentration between 0.1 and 25% w/v, preferably between 0.2 and 20% w/v, more preferably between 0.5 and 15% w/v, and
wherein LAP is contained in a concentration between 0.01 and 1 % w/v, preferably 0.02 and 0.5% w/v, more preferably 0.03 and 0.1% w/v.

2. The aqueous solution according to claim 1, wherein the at least one photo-curable polysaccharide has a molecular weight of between 30 kDa and 5 MDa, preferably between 40 kDa and 2.5 MDa, more preferably between 50 kDa and 1.25 MDa.

3. The aqueous solution according to any one of the preceding claims, wherein 3 to 70%, preferably 5 to 50%, more preferably 10 to 30% of the saccharide units of the at least one photo-curable polysaccharide exhibit a photo-curable group, preferably, wherein the photo-curable group is an acrylic group or a derivative thereof such as a methacrylic group.

4. The aqueous solution according to any one of the preceding claims, wherein the at least one photo-curable polysaccharide is a photo-curable hyaluronic acid, preferably, wherein the at least one photo-curable polysaccharide is acrylated and/or methacrylated hyaluronic acid.

5. The aqueous solution according to any one of the preceding claims, further comprising at least one glycosidase, preferably, wherein the at least one glycosidase is selected from the group consisting of chondroitinase ABC, chondroitinase AC, chondroitinase B, chondroitin 4-sulfatase, chondroitin 6-sulfatase, hyaluronidase and B-glucuronidase, and wherein the at least one glycosidase is contained in a concentration of between 1 to 5000 U/ml, preferably between 10 and 1000 U/ml, more preferably between 100 and 600 U/ml.

6. The aqueous solution according to any one of the preceding claims, wherein the aqueous solution is prepared for cross-linkage with a light source of between 250 nm and 1600 nm wavelength, preferably between 300 nm and 850 nm, more preferably between 350 nm and 650 nm, preferably, wherein the aqueous solution is prepared for cross-linkage with a light source at a power of between 0.1 and 100 mW/cm² and an exposure time of between 0.1 seconds and 30 minutes.

## Patentansprüche

1. Wässrige Lösung, umfassend:
(i) mindestens ein lichthärtbares Polysaccharid, und
(ii) Lithiumphenyl-2,4,6-trimethylbenzoylphosphinat (LAP) als Photoinitiator,
wobei das mindestens eine lichthärtbare Polysaccharid in einer Konzentration zwischen 0,1 und 25 % Gew./Vol., vorzugsweise zwischen 0,2 und 20 % Gew./Vol., mehr bevorzugt zwischen 0,5 und 15 % Gew./Vol. enthalten ist, und
wobei LAP in einer Konzentration zwischen 0,01 und 1 % Gew./Vol., vorzugsweise 0,02 und 0,5 % Gew./Vol., mehr bevorzugt 0,03 und 0,1 % Gew./Vol. enthalten ist.

2. Die wässrige Lösung nach Anspruch 1, wobei das mindestens eine lichthärtbare Polysaccharid ein Molekulargewicht zwischen 30 kDa und 5 MDa, vorzugsweise zwischen 40 kDa und 2,5 MDa, mehr bevorzugt zwischen 50 kDa und 1,25 MDa hat.

3. Die wässrige Lösung nach einem der vorhergehenden Ansprüche, wobei 3 bis 70 %, vorzugsweise 5 bis 50 %, mehr bevorzugt 10 bis 30 % der Saccharideinheiten des mindestens einen lichthärtbaren Polysaccharids eine lichthärtbare Gruppe aufweisen, vorzugsweise wobei die lichthärtbare Gruppe eine Acrylgruppe oder ein Derivat davon wie eine Methacrylgruppe ist.

4. Die wässrige Lösung nach einem der vorhergehenden Ansprüche, wobei das mindestens eine lichthärtbare Polysaccharid eine lichthärtbare Hyaluronsäure ist, vorzugsweise wobei das mindestens eine lichthärtbare Polysaccharid acrylierte und/oder methacrylierte Hyaluronsäure ist.

5. Die wässrige Lösung nach einem der vorhergehenden Ansprüche, ferner umfassend mindestens eine Glycosidase, vorzugsweise wobei die mindestens eine Glycosidase ausgewählt ist aus der Gruppe bestehend aus Chondroitinase ABC, Chondroitinase AC, Chondroitinase B, Chondroitin-4-sulfatase, Chondroitin-6-sulfatase, Hyaluronidase und B-Glucuronidase, und wobei die mindestens eine Glycosidase in einer Konzentration zwischen 1 und 5000 U/ml, vorzugsweise zwischen 10 und 1000 U/ml, mehr bevorzugt zwischen 100 und 600 U/ml enthalten ist.

6. Die wässrige Lösung nach einem der vorhergehenden Ansprüche, wobei die wässrige Lösung vorbereitet wird zur Vernetzung mit einer Lichtquelle mit einer Wellenlänge zwischen 250 nm und 1600 nm, vorzugsweise zwischen 300 nm und 850 nm, mehr bevorzugt zwischen 350 nm und 650 nm, vorzugsweise wobei die wässrige Lösung vorbereitet wird zur Vernetzung mit einer Lichtquelle mit einer Leistung zwischen 0,1 und 100 mW/cm² und einer Belichtungszeit zwischen 0,1 Sekunden und 30 Minuten.

## Revendications

1. Solution aqueuse comprenant :
(i) au moins un polysaccharide photodurcissable, et
(ii) du phényl-2,4,6-triméthylbenzoylphosphinate de lithium (LAP) en tant que photoamorceur,
dans laquelle l'au moins un polysaccharide photodurcissable est contenu en une concentration comprise entre 0,1 et 25 % p/v, de préférence entre 0,2 et 20 % p/v, plus préférentiellement entre 0,5 et 15 % p/v, et
dans laquelle le LAP est contenu en une concentration comprise entre 0,01 et 1 % p/v, de préférence entre 0,02 et 0,5 % p/v, plus préférentiellement entre 0,03 et 0,1 % p/v.

2. Solution aqueuse selon la revendication 1, dans laquelle l'au moins un polysaccharide photodurcissable a une masse moléculaire comprise entre 30 kDa et 5 MDa, de préférence entre 40 kDa et 2,5 MDa, plus préférentiellement entre 50 kDa et 1,25 MDa.

3. Solution aqueuse selon l'une des revendications précédentes, dans laquelle 3 à 70 %, de préférence 5 à 50 %, plus préférentiellement 10 à 30 %, des motifs saccharides de l'au moins un polysaccharide photodurcissable comprend un groupe photodurcissable, de préférence dans laquelle le groupe photodurcissable est un groupe acrylique ou un dérivé de celui-ci, tel qu'un groupe méthacrylique.

4. Solution aqueuse selon l'une des revendications précédentes, dans laquelle l'au moins un polysaccharide photodurcissable est un acide hyaluronique photodurcissable, de préférence dans laquelle l'au moins un polysaccharide photodurcissable est l'acide hyaluronique acrylé et/ou méthacrylé.

5. Solution aqueuse selon l'une des revendications précédentes, comprenant en outre au moins une glycosidase, de préférence dans laquelle l'au moins une glycosidase est choisie dans le groupe consistant en la chondroïtinase ABC, la chondroïtinase AC, la chondroïtinase B, la chondroïtine 4-sulfatase, la chondroïtine 6-sulfatase, l'hyaluronidase et la B-glucuronidase, et dans laquelle l'au moins une glycosidase est contenue en une concentration comprise entre 1 et 5 000 U/ml, de préférence entre 10 et 1 000 U/ml, plus préférentiellement entre 100 et 600 U/ml.

6. Solution aqueuse selon l'une des revendications précédentes, dans laquelle la solution aqueuse est préparée pour une réticulation avec une source de lumière ayant une longueur d'onde comprise entre 250 nm et 1 600 nm, de préférence entre 300 nm et 850 nm, plus préférentiellement entre 350 nm et 650 nm, la solution aqueuse étant de préférence préparée pour une réticulation avec une source de lumière présentant une puissance comprise entre 0,1 et 100 mW/cm² et un temps d'exposition compris entre 0,1 seconde et 30 minutes.
